**Europäisches Patentamt**

**European Patent Office**

**Office européen des brevets**

(19)

(11) Veröffentlichungsnummer : **0 204 776 B1**

(12) **EUROPÄISCHE PATENTSCHRIFT**

(45) Veröffentlichungstag der Patentschrift :
**01.02.89**

(21) Anmeldenummer : **86900049.7**

(22) Anmeldetag : **29.11.85**

(86) Internationale Anmeldenummer :
**PCT/DE 85/00505**

(87) Internationale Veröffentlichungsnummer :
**WO/86031 (05.06.86 Gazettee 86/12)**

(51) Int. Cl.⁴ : **A 61 F   2/38**

(54) **KNIEGELENKENDOPROTHESE.**

(30) Priorität : **29.11.84 DE 3444001**

(43) Veröffentlichungstag der Anmeldung :
**17.12.86 Patentblatt 86/51**

(45) Bekanntmachung des Hinweises auf die Patenterteilung : **01.02.89 Patentblatt 89/05**

(84) Benannte Vertragsstaaten :
**AT BE CH FR GB IT LI LU NL SE**

(56) Entgegenhaltungen :
**CH--A-- 267 810
DE--A- 3 314 038
FR--A- 2 330 377
FR--A- 2 378 503
GB--A- 2 067 412
US--A- 4 207 627**

(73) Patentinhaber : **S + G IMPLANTS GMBH
Grapengiesserstrasse 21
D-2400 Lübeck (DE)**

(72) Erfinder : **Hanslik, Lothar, Prof., Dr.
Leo-Baeck-Strasse 23
D-1000 Berlin 37 (DE)**
Erfinder : **Henssge, Ernst-Joachim, Prof., Dr.
Im Trentsaal 7
D-2400 Lübeck 1 (DE)**

(74) Vertreter : **Wilcken, Thomas, Dipl.-Ing. et al
Patentanwälte Wilcken & Weiss Musterbahn 1
D-2400 Lübeck1 (DE)**

**Beschreibung**

Die Erfindung bezieht sich auf eine Kniegelenkendoprothese, bestehend aus einem Oberschenkelteil, das mit zwei Kufenflächen versehen ist, die zwischen sich einen Raum freilassen, vorn miteinander verbunden sind, deren Krümmung von vorn nach hinten zunimmt und die in Ebenen senkrecht zu dieser Krümmung ebenfalls gekrümmt sind, aus einem Schienbeinteil, das zwei Flächen aufweist, auf denen die Kufenflächen gleitend abrollen und zwischen denen sich eine Erhöhung befindet, und aus künstlichen Verbindungsmitteln, die die beiden Prothesenteile zusammenhalten.

Bei einer bekannten Prothese dieser Art (DE-A-25 49 819) sind die beiden Flächen des Schienbeinteils in jeweils einer mittleren Schnittebene von vorn nach hinten stark konkav, d. h. den Gleitflächen des Oberschenkelteils kongruent angepaßt. Die Abhänge von der Erhöhung zu den beiden Flächen sind gleich, und die Prothesenteile werden über zwei Zapfen in der Erhöhung und zwei Nuten in den Kufen, in die die Zapfen eingreifen, zusammengehalten. Der Nachteil dieses Gelenkes besteht darin, daß die Bewegungsverhältnisse nur sehr unvollkommen den Bewegungsverhältnissen bei einem natürlichen Gelenk entsprechen.

Der Erfindung liegt die Aufgabe zugrunde, die eingangs erwähnte Prothese derart auszubilden, daß die Bewegungsverhältnisse besser als im bekannten Fall den natürlichen Bewegungsverhältnissen entsprechen und Verschleißerscheinungen vermieden werden.

Diese Aufgabe wird erfindungsgemäß dadurch gelöst, daß die äußere Fläche des Schienbeinteils in einer mittleren Schnittebene von vorn nach hinten weitgehend eben ist, daß die innere Fläche des Schienbeinteils in einer mittleren Schnittebene von vorn nach hinten leicht konkav ist, daß der Abhang der Erhöhung zur äußeren Fläche flacher als der Abhang der Erhöhung zur inneren Fläche ist und daß die Prothesenteile durch zwei endliche, unelastische Bänder zusammengehalten werden, von denen ein erstes Band von der Mitte vorn des Schienbeinteils nach oben, außen und schräg nach hinten zum Oberschenkelteil verläuft und von denen ein zweites Band von der Mitte hinten des Schienbeinteils nach oben, innen und schräg nach vorn zum Oberschenkelteil verläuft, wobei die Bänder bei gestrecktem Knie gespannt und bei gebeugtem Knie im unbelasteten Zustand leicht entspannt sind.

Auf diese Weise wird folgendes erreicht : Beim Beugen des Gelenkes überstreicht der Berührungsbereich der inneren Kufe auf der ihm zugeordneten Fläche einen kleinen Bogen, während der Berührungsbereich der äußeren Kufe auf der ihm zugeordneten Fläche einen großen Bogen überstreicht. Das entspricht den natürlichen Verhältnissen.

Bezüglich der Spannung der Bänder gilt folgendes : Sie sind bei gestrecktem Knie gespannt und bei gebeugtem Knie im unbelasteten Zustand leicht entspannt. Im belasteten, gebeugten Zustand sind beide Bänder gespannt. Beim Beugen des Knies im unbelasteten Zustand aus der gestreckten Lage heraus nimmt die Spannung der Bänder allmählich ab.

Selbstverständlich müssen die Punkte, wo die Bänder mit den Prothesenteilen verbunden sind, so liegen, daß die in Frage kommenden Bewegungen nicht behindert werden. Das von vorn unten nach oben, außen verlaufende Band darf beispielsweise nicht zu weit vorn mit dem Schienbeinteil verbunden sein. Da die Krümmungen der Kufen des Oberschenkelteils nach hinten abnehmen, ist dann aber die einwandfreie Beugung des Gelenkes gewährleistet.

Eine Weiterentwicklung der Erfindung besteht darin, daß zwei Erhöhungen zwischen den Flächen vorgesehen sind, wobei die äußere etwas hinter der inneren liegt, und diese über eine Sattelfläche miteinander verbunden sind. Auf diese Weise wird erreicht, daß der mittlere Teil des Schienbeinteils den verschiedenen Bänderlagen in den verschiedenen Winkellagen des Knies angepaßt ist.

Weiterhin wird vorgeschlagen, daß der Abhang zur äußeren Fläche allmählich kegelförmig in die Fläche übergeht.

Sodann wird vorgeschlagen, daß die äußere Fläche des Schienbeinteils höher als die innere Fläche des Schienbeinteils ist, bezogen auf den Boden, auf dem der Mensch steht. Analoges gilt dann für die beiden Kufenflächen des Oberschenkelteils. Für diese Ausbildung ist insbesondere die Tatsache maßgeblich, daß die Beine des Menschen, von vorn gesehen, annähernd ein « X·» bilden.

Eine Weiterentwicklung der Erfindung besteht ferner darin, daß die innere und äußere Fläche im hinteren Bereich nach hinten abfallen. Dadurch wird erreicht, daß das Oberschenkelteil im stark gebeugten Zustand auf diesen beiden Abhängen gleiten bzw. abrollen kann, was die Beugebewegung des Knies erleichtert.

Schließlich wird vorgeschlagen, daß die Bänder vor dem Einsetzen der Prothese in das menschliche Bein mit dem Schienbeinteil fest verbunden sind und während des Einsetzens mit dem Oberschenkelteil bzw. Oberschenkelknochen verbunden werden.

Weitere Einzelheiten der Erfindung ergeben sich aus der Zeichnung. Darin zeigen :

Fig. 1 eine Draufsicht auf eine Ausführungsform eines erfindungsgemäßen Schienbeinprothesenteils.

Fig. 2 ebenfalls eine Draufsicht auf dieses Teil und

Fig. 3 einen Schnitt nach der Linie III-III der Fig. 2.

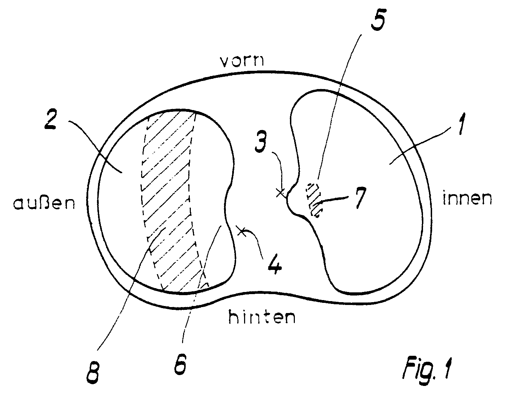

In Fig. 1 ist das Schienbeinprothesenteil von oben gesehen dargestellt. Die innere Gleitfläche für das Oberschenkelprothesenteil ist mit 1, die äußere Gleitfläche für das Oberschenkelprothesenteil ist mit 2 bezeichnet. Zwischen den beiden Flächen 1 und 2 befinden sich zwei Erhöhungen, deren Gipfel mit 3 und 4 bezeichnet sind. Der Abhang von der Erhöhung 3 in die Fläche 1, der mit 5 bezeichnet ist, ist steiler als der Abhang von der Erhöhung 4 in die Fläche 2, der mit 6 bezeichnet ist. Die Gleitfläche 1 ist leicht konkav, während die Gleitfläche 2 in etwa eben ist. Die Folge der erwähnten Formgebungen ist die, daß die beiden Kufen des Oberschenkelteils beim Beugen des Knies Flächen berühren, die mit 7 und 8 bezeichnet sind und in der Fig. 1 schraffiert dargestellt sind. Die innere Kufe überstreicht also einen kleinen Bereich 7, während die äußere Kufe einen großen Bereich 8 überstreicht. Stellt man sich das Schienbein fest vor und nimmt an, daß der Oberschenkel gegenüber dem Schienbein gebeugt wird, dann vollführt der Oberschenkel während der Beugung eine leichte Schwenkung im Gegenuhrzeigersinn (bezogen auf Fig. 1). Anders ausgedrückt ist es so, daß bei festem Oberschenkel das Schienbein bei der Beugung eine solche Schwenkung um seine Längsachse vollführt, daß ein vorderer Punkt des Schienbeins sich etwas nach innen dreht. Die Verhältnisse sollten solcher Art sein, daß bei einem Beugungswinkel des Knies von 90° der Schwenkwinkel des Schienbeins um seine Längsachse ca. 20° beträgt.

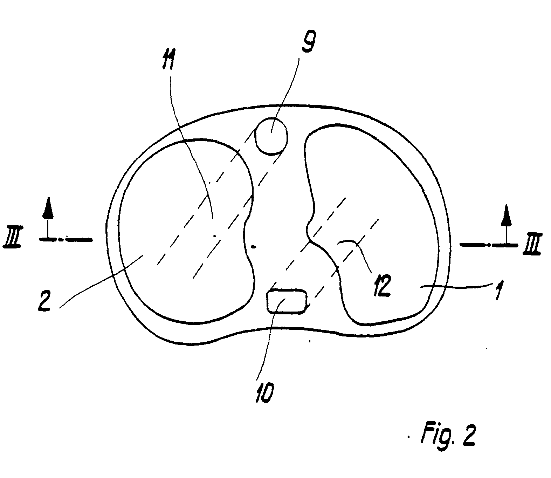

Fig. 2 zeigt abermals eine Draufsicht des Schienbeinprothesenteils. In Fig. 2 sind die beiden Bänder eingezeichnet und mit 11 und 12 bezeichnet. Sie sind bei 9 und 10 mit dem Schienbeinteil verbunden und laufen dann nach oben (siehe die strichpunktierten Linien 11 und 12) in das Oberschenkelteil.

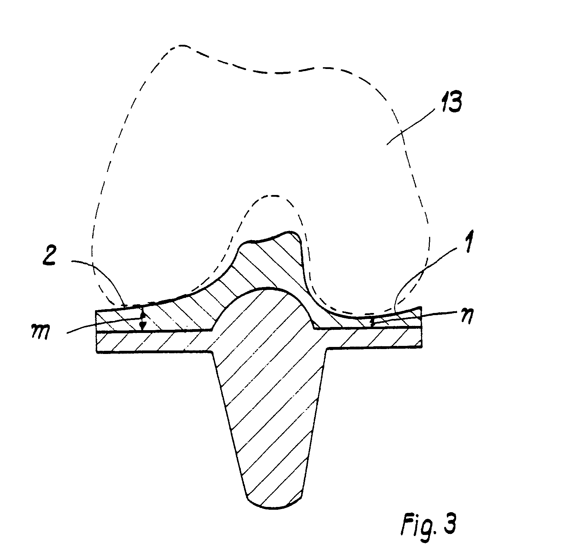

Fig. 3 zeigt einen Schnitt nach der Linie III-III der Fig. 2 durch das Schienbeinprothesenteil. Man erkennt, daß die Fläche 2 höher ist als die Fläche 1 (der Abstand m ist also größer als der Abstand n).

Das Oberschenkelteil ist gestrichelt dargestellt und mit 13 bezeichnet. Es gilt für vorne und hinten :

$$1{,}48 < m/n < 1{,}52$$

insbesondere $m/n = 1{,}5$
und für die Mitte :

$$1{,}98 < m/n < 2{,}02$$

insbesondere $m/n = 2$.

## Patentansprüche

1. Kniegelenkendoprothese, bestehend aus einem Oberschenkelteil (13), das mit zwei Kufenflächen versehen ist, die zwischen sich einen Raum freilassen, vorn miteinander verbunden sind, deren Krümmung von vorn nach hinten zunimmt und die in Ebenen senkrecht zu dieser Krümmung ebenfalls gekrümmt sind, aus einem Schienbeinteil, das zwei Flächen (1, 2) aufweist, auf denen die Kufenflächen gleitend abrollen und zwischen denen sich eine Erhöhung befindet, und aus künstlichen Verbindungsmitteln (11, 12), die die beiden Prothesenteile zusammenhalten, dadurch gekennzeichnet, daß die äußere Fläche (2) des Schienbeinteils in einer mittleren Schnittebene von vorn nach hinten weitgehend eben ist, daß die innere Fläche (1) des Schienbeinteils in einer mittleren Schnittebene von vorn nach hinten leicht konkav ist, daß der Abhang (6) der Erhöhung (3, 4) zur äußeren Fläche (2) flacher als der Abhang (5) der Erhöhung (3, 4) zur inneren Fläche (1) ist und daß die Prothesenteile durch zwei endliche, unelastische Bänder (11, 12) zusammengehalten werden, von denen ein erstes Band (11) von der Mitte vorn des Schienbeinteils nach oben, außen und schräg nach hinten zum Oberschenkelteil verläuft und von denen ein zweites Band (12) von der Mitte hinten des Schienbeinteils nach oben, innen und schräg nach vorn zum Oberschenkelteil verläuft, wobei die Bänder (11, 12) bei gestrecktem Knie gespannt und bei gebeugtem Knie im unbelasteten Zustand leicht entspannt sind.

2. Kniegelenkendoprothese nach Anspruch 1, dadurch gekennzeichnet, daß zwei Erhöhungen (3, 4) zwischen den Schienbeinteilflächen (1, 2) vorgesehen sind, wobei die äußere (4) etwas hinter der inneren (3) liegt und die über eine Sattelfläche miteinander verbunden sind.

3. Kniegelenkendoprothese nach Anspruch 1 oder 2, dadurch gekennzeichnet, daß der Abhang (6) zur äußeren Fläche (2) allmählich kegelförmig in die Fläche (2) übergeht.

4. Kniegelenkendoprothese nach einem der Ansprüche 1 bis 3, dadurch gekennzeichnet, daß die äußere Fläche (2) des Schienbeinteils höher als die innere Fläche des Schienbeinteils ist, bezogen auf den Boden, auf dem der Mensch steht.

5. Kniegelenkendoprothese nach Anspruch 4, dadurch gekennzeichnet, daß die Kufenfläche, die der äußeren Schienbeinteilfläche (2) anliegt, höher ist als die andere Kufenfläche, bezogen auf den Boden, auf dem der Mensch steht.

6. Kniegelenkendoprothese nach einem der Ansprüche 1 bis 5, dadurch gekennzeichnet, daß die innere und äußere Schienbeinteilfläche (1, 2) im hinteren Bereich nach hinten abfallen.

7. Kniegelenkendoprothese nach einem der Ansprüche 1 bis 6, dadurch gekennzeichnet, daß die Bänder (11, 12) vor dem Einsetzen der Prothese in das menschliche Bein mit dem Schienbeinteil fest verbunden sind und während des Einsetzens mit dem Oberschenkelteil bzw. Oberschenkelknochen verbunden werden.

## Claims

1. A knee joint endoprosthesis, comprising a thigh part (13) which is provided with two runner surfaces which have a free space between them, are interconnected at the front, the curvature of which increases from the front towards the rear and which are equally curved in planes at right angles to this curvature, a tibia part which has two surfaces (1, 2) on which the runner surfaces slidingly roll along and between which is situated a protuberance, and artificial connecting means (11, 12) which hold together the two prosthesis parts, characterised in that the outer surface (2) of the tibia part is largely planar in a central sectional plane from the front towards the rear, that the inner surface (1) of the tibia part is slightly concave in a central sectional plane from the front towards the rear, that the slope (6) of the protuberance (3, 4) towards the outer surface (2) is shallower than the slope (5) of the protuberance (3, 4) towards the inner surface (1) and that the prosthesis parts are held together by means of two terminal inelastic straps (11, 12), a first strap (11) of which extends from the front centre of the tibia part, upwards, outwards and rearwards obliquely to the thigh part, and a second strap (12) of which extends from the rear centre of the tibia part, upwards, inwards and forwards obliquely to the thigh part, the straps (11, 12) being tensioned when the knee is flattened, and slackened to a slight extent when the knee is bent in an unencumbered position.

2. A knee joint endoprosthesis according to claim 1, characterised in that two protuberances (3, 4) are provided between the tibia part surfaces (1, 2), the outer (4) being situated a little behind the inner (3) and the two being joined together via a saddle surface.

3. A knee joint endoprosthesis according to claim 1 or 2, characterised in that the slope (6) towards the outer surface (2) gradually merges conically into the surface (2).

4. A knee joint endoprosthesis according to one of the claims 1 to 3, characterised in that the outer surface (2) of the tibia part is higher than the inner surface of the tibia part with respect to the floor which is stood upon by the person.

5. A knee joint endoprosthesis according to claim 4, characterised in that the runner surface which is in contact with the outer tibia part surface (2) is higher than the other runner surface with respect to the floor which is stood upon by the person.

6. A knee joint endoprosthesis according to one of the claims 1 to 5, characterised in that the inner and outer tibia part surfaces (1, 2) slope down rearwards in the rear portion.

7. A knee joint endoprosthesis according to one of the claims 1 to 6, characterised in that the straps (11, 12) are fixedly joined to the tibia part prior to the insertion of the prosthesis into the human leg, and are joined to the thigh part or femur during the said insertion.

## Revendications

1. Endoprothèse de l'articulation du genou, constituée par une partie de cuisse (13), qui est munie de deux surfaces formant patins, qui ménagent entre elles un espace et qui sont reliées entre elles sur l'avant et dont la courbure augmente de l'avant vers l'arrière, ces surfaces étant également cintrées dans des plans perpendiculaires à cette courbure, par une partie de tibia, qui possède deux surfaces (1, 2), sur lesquelles les surfaces formant patins roulent en glissant et entre lesquelles se trouve située une partie surélevée, et par des moyens prothétiques de liaison (11, 12), qui maintiennent à l'état réuni les deux parties de la prothèse, caractérisée en ce que la surface extérieure (2) de l'élément de tibia est dans une large mesure plate de l'avant vers l'arrière, dans un plan de coupe médian, que la surface intérieure (1) de la partie de tibia est légèrement concave de l'avant vers l'arrière dans un plan de coupe médian, que la zone (6) de la partie surélevée (3, 4), en direction de la surface extérieure (2), est plus aplatie que la zone (5) de la partie surélevée (3, 4) en direction de la surface intérieure (1) et que les parties de la prothèse sont maintenues assemblées par deux bandes de longueur finie non élastiques (11, 12), dont la première (11) s'étend depuis la zone centrale à l'avant de la partie de tibia vers le haut, en direction de l'extérieur et obliquement vers l'arrière en direction de la partie de cuisse et dont la seconde (12) s'étend depuis la zone centrale à l'arrière de la partie de tibia vers le haut, en direction de l'intérieur et obliquement vers l'avant en direction de la partie de cuisse, les bandes (11, 12) étant tendues lorsque le genou est étendu et sont légèrement détendues, en étant déchargées de toute contrainte, lorsque le genou est plié.

2. Endoprothèse de l'articulation du genou selon la revendication 1, caractérisée en ce que deux parties surélevées (3, 4) sont prévues entre les surfaces (1, 2) de la partie du tibia, la partie surélevée extérieure (4) étant située légèrement en arrière de la partie surélevée intérieure (3) et ces deux parties étant reliées entre elles par l'intermédiaire d'une surface en forme de selle.

3. Endoprothèse de l'articulation du genou selon la revendication 1 ou 2, caractérisée en ce que la zone (6), en direction de la surface extérieure (2), se transforme progressivement avec une forme conique pour former la surface (2).

4. Endoprothèse de l'articulation du genou selon l'une des revendications 1 à 3, caractérisée en ce que la surface extérieure (2) de la partie de tibia est plus haute que la surface intérieure de la partie de tibia, par rapport au sol, sur lequel la personne se tient debout.

5. Endoprothèse de l'articulation du genou selon la revendication 4, caractérisée en ce que la surface formant patin, qui s'applique sur la surface partielle extérieure (2) du tibia, est plus haute que l'autre surface formant patin, par rapport au sol, sur lequel la personne se tient debout.

6. Endoprothèse de l'articulation du genou selon l'une des revendications 1 à 5, caractérisée en ce que les surfaces partielles intérieure et extérieure (1, 2) du tibia s'abaissent vers l'arrière, dans la zone arrière.

7. Endoprothèse de l'articulation du genou selon l'une des revendications 1 à 6, caractérisée en ce que les bandes (11, 12) sont reliées rigidement à l'élément de cuisse avant l'insertion de la prothèse dans la jambe humaine et sont reliées à la partie de cuisse ou au fémur pendant son insertion.

vorn

2

außen

3

1

innen

5

7

4

8

6

hinten

*Fig. 1*

Fig. 2

Fig. 3